# FASCICULE DE BREVET EUROPEEN

(11) **EP 1 093 809 B1**
(45) Date de publication et mention de la délivrance du brevet: **25.10.2006**
(21) Numéro de dépôt: 00402821.3
(22) Date de dépôt: 12.10.2000
(51) Int. Cl.: A61K 8/891, A61Q 5/02, A61Q 19/10, A61Q 5/12

(54) **Compositions cosmétiques contenant un copolymère vinyldiméthicone/diméthicone et un agent conditionneur et leurs utilisations**
Kosmetische Zusammensetzungen enhaltend ein Vinyldimethicon/Dimethicon Copolymer und ein Konditionierungsmittel, und ihre Anwendungen
Cosmetic compositions containing a vinyldimethicone/dimethicone copolymer and a conditioning agent, and their uses

(30) Priorité: 20.10.1999 FR 9913097
(43) Date de publication de la demande: 25.04.2001
(73) Titulaire: L'ORÉAL, 75008 Paris (FR)
(72) Inventeur: Decoster, Sandrine, 95210 Saint Gratien (FR); Douin, Véronique, 75017 Paris (FR); Bailly, Virginie, 92110 Clichy (FR)
(74) Mandataire: Le Blainvaux Bellegarde, Françoise

(56) Documents cités:
- EP-A- 0 787 758
- EP-A- 0 874 017
- FR-A- 2 739 284
- FR-A- 2 742 657
- FR-A- 2 748 392
- FR-A- 2 755 854
- FR-A- 2 770 523
- US-A- 5 679 357

## Description

La présente invention concerne de nouvelles compositions cosmétiques comprenant dans un milieu cosmétiquement acceptable au moins un agent conditionneur particulier et au moins un copolymère dimethicone à insaturation éthylénique/dimethicone particulier.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).
En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

En résumé, il s'avère que les compositions cosmétiques actuelles contenant des agents conditionneurs, ne donnent pas complètement satisfaction.

La demanderesse a maintenant découvert que l'association d'un copolymère siliconé défini ci-dessous de viscosité comprise entre 10⁶ et 100.10⁶ cP avec certains agents conditionneurs permet de remédier à ces inconvénients.

Ainsi, à la suite d'importantes recherches menées sur la question, il a maintenant été trouvé par la Demanderesse qu'en introduisant un copolymère siliconé défini ci-dessous de viscosité comprise entre 10⁶ et 100.10⁶ cP dans les compositions en particulier capillaires de l'art antérieur à base d'agents conditionneurs, il est possible de limiter, voire supprimer, les problèmes généralement liés à l'emploi de telles compositions, à savoir en particulier l'alourdissement (toucher chargé lors d'applications répétées), le manque de lissage et de douceur des cheveux, tout en conservant les autres propriétés cosmétiques avantageuses qui sont attachés aux compositions à base d'agents conditionneurs.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un copolymère siliconé défini ci-dessous de viscosité comprise entre 10⁶ et 100.10⁶ cP et au moins un agent conditionneur choisi parmi :
- les huiles de synthèse
- les huiles animales ou végétales et
- les huiles fluorées ou perfluorées,
- les cires naturelles ou synthétiques
- les céramides de formule (I) suivante : 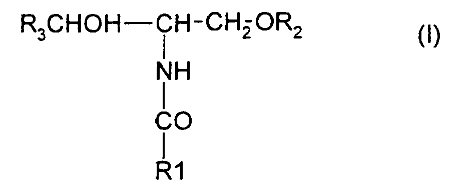
dans laquelle :
- R₁ désigne un radical alkyle, linéaire ou ramifié, saturé ou insaturé, dérivé d'acides gras en C₁₄-C₃₀, ce radical pouvant être substitué par un groupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un radical hydrocarboné en C₁₅-C₂₆, saturé ou insaturé en position α , ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀.

Un autre objet de l'invention concerne l'utilisation d'un copolymère siliconé défini ci-dessous de viscosité comprise entre 10⁶ et 100.10⁶ cP dans, ou pour la fabrication d'une composition cosmétique comprenant un agent conditionneur tel que défini ci-dessus.

Les différents objets de l'invention vont maintenant être détaillés. L'ensemble des significations et définitions des composés utilisés dans la présente invention données ci-dessous sont valables pour l'ensemble des objets de l'invention.

Dans le cadre de la présente demande, on entend par agent conditionneur tout agent ayant pour fonction l'amélioration des propriétés cosmétiques des cheveux, en particulier la douceur, le démêlage, le toucher, l'électricité statique.

Le copolymère siliconé résulte de la réaction d'addition, en présence d'un catalyseur, d'au moins :
- (a) un polysiloxane de formule (I): 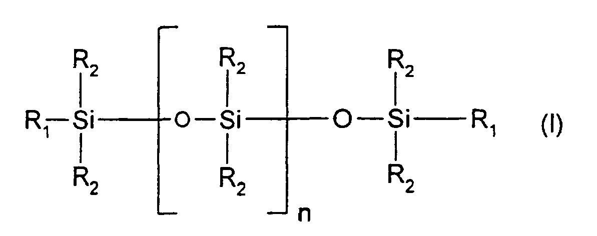 dans laquelle :
   R1 désigne un groupement susceptible de réagir par réaction d'addition de chaîne tel que par exemple un atome d'hydrogène, un groupement aliphatique de préférence en C2-C6 ayant une insaturation éthylénique notamment vinyl, allyl ou héxényl,
   Les groupements R2 de la formule (I) peuvent représenter notamment des groupements alkyle, cycloalkyle, aryle, alkylaryle ou hydroxyle, et peuvent comporter en outre, des groupements fonctionnels tels qu'éthers, amines, carboxyles, hydroxyles, thiols, esters, sulfonates, sulfates.
   Les groupements alkyle ont par exemple 1 à 20 atomes de carbone ; les groupements cycloalkyle ont par exemple 5 ou 6 atomes de carbone ; les groupements aryle sont notamment des groupements phényle ;les groupements alkylaryle peuvent avoir de 7 à 20 atomes de carbone.
   Plus particulièrement R2 désigne méthyle.
   n est un entier tel que le polysiloxane de formule (I) ait de préférence une viscosité cinématique comprise entre 1 et 1.10⁶ mm²/s..n varie notamment de 5 à 5000.
- (b) et d'au moins un composé siliconé comprenant au moins un et au plus deux groupements susceptibles de réagir avec les groupements R1 du polysiloxane (a), l'un au moins des composés de type (a) ou (b) contient un groupe aliphatique ayant une insaturation éthylénique.

Les composés de type (b) sont un autre polysiloxane de type (a) dans lequel les groupements R1 du polysiloxane (b) sont susceptibles de réagir avec les groupement R1 du polysiloxane (a).

De préférence, les copolymères siliconés sont notamment obtenus par réaction d'addition, en présence d'un catalyseur d'hydrosilylation (par exemple un catalyseur au platine), d'au moins :
- (a) un alpha,oméga-di vinyl polydiméthylsiloxane, et
- (b) d'un alpha,oméga-di hydrogéno polydiméthylsiloxane.

Le copolymère a généralement une viscosité dynamique, mesurée à la température d'environ 25°C et au taux de cisaillement de 0,01Hz pour une contrainte de 1500 Pa, comprise entre 10⁶ et 100.10⁶ cP et de préférence comprise entre 5.10⁶ cP et 30.10⁶ cP.

Toutes les mesures de viscosités dynamiques données dans la présente demande ont été effectuées à une température d'environ 25°C, sur un Carri-Med CSL2-500.

La viscosité cinématique est par exemple mesurée à 25°C selon la norme ASTM 445 Appendice C.

Les copolymères siliconés selon l'invention sont essentiellement non réticulés.

Le copolymère siliconé présent dans la composition selon l'invention peut se présenter sous la forme d'émulsion aqueuse.
Par émulsion aqueuse, on entend une émulsion de type huile-dans-eau dans laquelle le copolymère siliconé est dispersé sous forme de particules ou de gouttelettes dans la phase aqueuse formant la phase continue de l'émulsion.
Cette émulsion peut être stabilisée par un système émulsionnant usuel.
Cette émulsion de silicone peut avoir une taille de gouttelettes ou de particules de silicone allant de 10 nm à 50 µm, et de préférence de 0,3 µm à 20 µm.
La taille des particules est mesurée par granulométrie laser.
Le système émulsionnant comprend des tensioactifs employés habituellement dans les émulsions de silicone. Ces tensioactifs peuvent être non-ioniques, cationiques, anioniques ou amphotères ou leurs mélanges tels que ceux décrits ci-dessous.

Le système émulsionnant représente de 0,5 % à 10 % en poids par rapport au poids total de l'émulsion.

La synthèse de ces émulsions de silicones est notamment décrite dans la demande EP-A-874017.

De telles émulsions sont notamment commercialisées sous la dénomination DC2-1997 cationic emulsion par la société DOW CORNING. Cette émulsion comprend un copolymère α,ω̅-divinyl diméthicone/α,ω̅-dihydrogénodiméthicone ayant une viscosité dynamique d'environ 15.10⁶ cP, un émulsionnant de type cationique tel que le chlorure de cétyltriméthylammonium, un stabilisant de type hydroxyéthylcellulose et de l'eau.

Le copolymère siliconé est présent de préférence en une concentration comprise entre 0,05 et 10% en poids par rapport au poids total de la composition. Plus préférentiellement, cette quantité est comprise entre 0,1 et 5% en poids par rapport au poids total de la composition.

Les agents de conditionnement peuvent se présenter sous forme liquide, semi-solide ou solide tels que par exemple des huiles, des cires ou des gommes.

Les huiles de synthèse sont notamment les polyoléfines en particulier les poly-α-oléfines et plus particulièrement :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation), .
- de type polydécène, hydrogéné ou non.
   De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol,
ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;
On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Selon la présente invention, les composés de type céramide sont notamment les céramides et/ou les glycocéramides et/ou les pseudocéramides et/ou les néocéramides, naturelles ou synthétiques de formule générale (I): 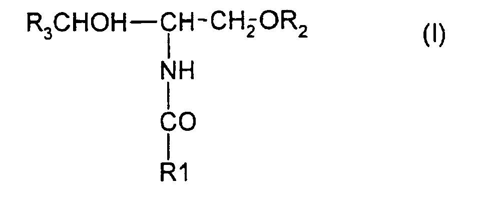 dans laquelle :
- R₁ désigne un radical alkyle, linéaire ou ramifié, saturé ou insaturé, dérivé d'acides gras en C₁₄-C₃₀, ce radical pouvant être substitué par un groupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un radical hydrocarboné en C₁₅-C₂₆, saturé ou insaturé en position α , ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀.

Les céramides plus particulièrement préférés selon l'invention sont les composés de formule (I) pour lesquels R₁ désigne un alkyle saturé ou insaturé dérivé d'acides gras en C₁₆-C₂₂ ; R₂ désigne un atome d'hydrogène ; et R₃ désigne un radical linéaire saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

Encore plus préférentiellement, on utilise les composés de formule (I) pour lesquels R₁ désigne un radical alkyle saturé ou insaturé dérivé d'acides gras ; R₂ désigne un radical galactosyle ou sulfogalactosyle ; et R₃ désigne un groupement

-CH=CH-(CH₂)₁₂-CH₃.

A titre d'exemple, on peut citer le produit constitué d'un mélange de ces composés, vendu sous la dénomination commerciale GLYCOCER par la société WAITAKI INTERNATIONAL BIOSCIENCES.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

Les huiles fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Il est bien entendu possible de mettre en oeuvre des mélanges d'agents conditionneurs.

Selon l'invention, le ou les agents conditionneurs peuvent représenter de 0,001 % à 10 % en poids, de préférence de 0,01 % à 5% en poids et plus particulièrement de 0,1 à 3% en poids par rapport au poids total de la composition finale.

Les compositions de l'invention peuvent en outre contenir avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, a-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀- C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈₋C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

Encore plus préférentiellement les compositions selon l'invention peuvent contenir en outre au moins un tensioactif cationique.
Les tensioactifs cationiques peuvent être choisis parmi :
A) les sels d'ammonium quaternaires de la formule générale (IV) suivante : 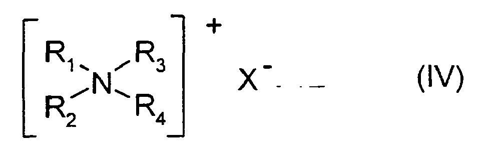 dans laquelle X est un anion choisi dans le groupe des halogénures (chlorure, bromure
   ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate. , et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (V) suivante : 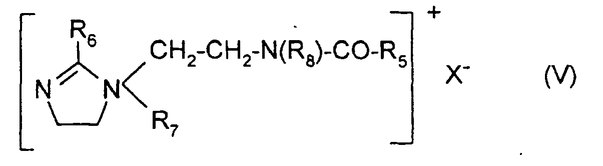
dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (VI) : 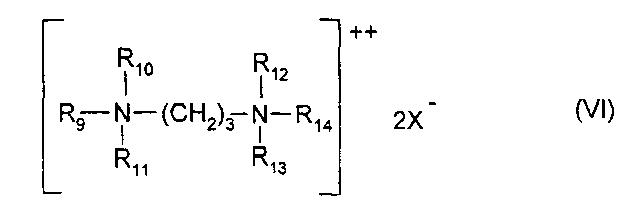 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène
   ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (VII) suivante : 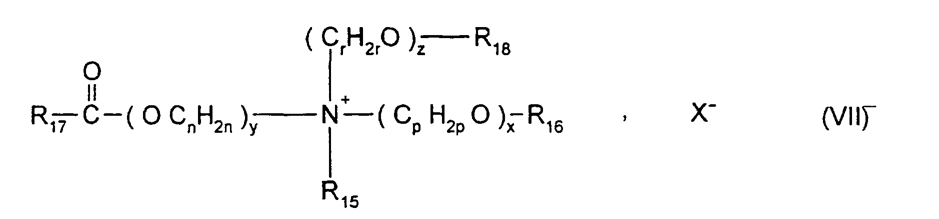 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
   - le radical 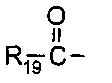
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 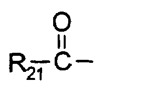
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 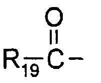
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 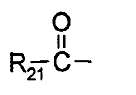
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Le tensioactif cationique est généralement présent dans des concentrations allant de 0,1 à 10% en poids par rapport au poids total de la composition et de préférence de 0,5 à 7 % en poids et plus préférentiellement entre 1 et 5% en poids.

La composition de l'invention peut également contenir au moins un additif choisi parmi les épaississants, les parfums, les agents nacrants, les conservateurs, les filtres solaires siliconés ou non, les vitamines, les provitamines, les polymères cationiques, amphotères, anioniques ou non ioniques, les protéines, les hydrolysats de protéine, l'acide méthyl-18 eicosanoique, les hydroxyacides, le panthénol, les silicones volatiles ou non volatiles, cycliques ou linéaires ou réticulés, modifiées ou non, et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions d'après-shampooing à rincer ou non.
Les compositions selon l'invention peuvent être des compositions détergentes telles que des shampooings, des gels-douche, des bains moussants et peuvent être également des démaquillants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques et cationiques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

Dans les exemples, les noms commerciaux ont les définitions suivantes :

### EXEMPLE 1

On a réalisé un après-shampooing conforme à l'invention de composition suivante :
- Mélange de myristate, palmitate et stéarate de
   myristyle, cétyle et stéaryle 0,5 g
- Chlorure de béhényl triméthyl ammonium
   (GENAMIN KDMP de CLARIANT) 1,2 g MA
- N-oléoyl dihydrosphingosine 0,01 g
- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 1,36 gMA
- Mélange d'alcool cétylique et d'alcool stéarylique
   (50/50 en poids) 3 g
- Méthosulfate de méthyl alkyl alkylamidoéthyl imidazolinium en solution à 75 % ma dans le propylène
   glycol (REWOQUAT W 75 PG de REWO) 0,05 gMA
- Lauryldiméthiconecopolyol à 91% de MA
   (Q2-5200 de DOW CORNING) 0,23 gMA
- Parfum, conservateurs qs
- Eau qsp 100 g

On applique cette composition sur des cheveux lavés et essorés. On laisse pauser pendant 2 minutes, puis on rince à l'eau.

Les cheveux traités avec cet après-shampooing sont doux, lisses et se démêlent facilement.

## Revendications

1. Composition cosmétique, **caractérisée par le fait qu'**elle comprend, dans un milieu cosmétiquement acceptable, au moins un agent conditionneur choisi parmi :
- les huiles de synthèse
- les huiles animales ou végétales et
- les huiles fluorées ou perfluorées,
- les cires naturelles ou synthétiques
- les céramides de formule (I) suivante : 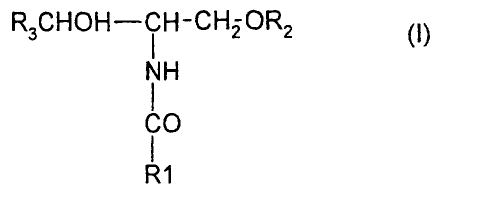 dans laquelle :
- R₁ désigne un radical alkyle, linéaire ou ramifié, saturé ou insaturé, dérivé d'acides gras en C₁₄-C₃₀, ce radical pouvant être substitué par un groupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un radical hydrocarboné en C₁₅-C₂₆, saturé ou insaturé en position α , ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀,
et
au moins un copolymère siliconé de viscosité comprise entre 10⁶ et 100.10⁶ cP résutant de la réaction d'addition, en présence d'un catalyseur, d'au moins :
- (a) un polysiloxane de formule (I) ; 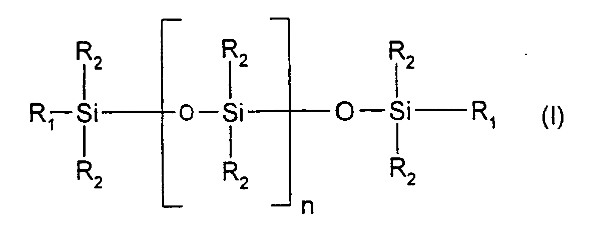 dans laquelle :
R1 désigne un groupement susceptible de réagir par réaction d'addition de chaîne tel que par exemple un atome d'hydrogène, un groupement aliphatique ayant une insaturation éthylénique notamment vinyl, allyl ou héxényl,
les groupements R2 de la formule (I) représentent des groupements alkyle ayant de 1 à 20 atomes de carbone, cycloalkyle ayant 5 ou 6 atomes de carbone, aryle, alkylaryle ayant de 7 à 20 atomes de carbone ou hydroxyle, et peuvent comporter en outre, des groupements fonctionnels tels qu'éthers, amines, carboxyles, hydroxyles, thiols, esters, sulfonates, sulfates,
n est un entier tel que le polysiloxane de formule (I) ait de préférence une viscosité cinématique comprise entre 1 et 1.10⁶ mm²/s..
- (b) et d'au moins un composé siliconé comprenant au moins un et au plus deux groupements susceptibles de réagir avec les groupements R1 du polysiloxane (a), l'un au moins des composés de type (a) ou (b) contient un groupe aliphatique ayant une insaturation éthylénique.

2. Composition selon la revendication 1, **caractérisée par le fait que** R2 désigne méthyle.

3. Composition selon l'une des revendications 1 ou 2, **caractérisée par le fait que** le composé de type (b) est un autre polysiloxane de type (a) dans lequel les groupements R1 du polysiloxane (b) sont susceptibles de réagir avec les groupement R1 du polysiloxane (a).

4. Composition selon l'une quelconque des revendications 1 à 3, **caractérisée par le fait que** le copolymère siliconé est obtenu par réaction d'addition, en présence d'un catalyseur d'hydrosilylation, d'au moins :
- (a) un alpha,oméga-di vinyl polydiméthylsiloxane, et
- (b) d'un alpha,oméga-di hydrogéno polydiméthylsiloxane.

5. Composition selon l'une quelconque des revendications 1 à 4, **caractérisée par le fait que** le copolymère siliconé est sous la forme d'émulsion aqueuse.

6. Composition selon l'une quelconque des revendications 1 à 5, **caractérisée par le fait que** le copolymère siliconé est présent à une concentration comprise entre 0,05 et 10% en poids par rapport au poids total de la composition.

7. Composition selon l'une quelconque des revendications 1 à 6, **caractérisée par le fait que** les huiles de synthèse sont des polyoléfines de type polybutène, hydrogéné ou non, ou de type polydécène, hydrogéné ou non.

8. Composition selon l'une quelconque des revendications 1 à 6, **caractérisée par le fait que** les huiles animales ou végétales sont choisies dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote.

9. Composition selon l'une quelconque des revendications 1 à 6, **caractérisée par le fait que** la cire ou les cires sont choisies parmi la cire de Carnauba, la cire de Candelila, la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis, les cires animales comme les cires d'abeilles, les cires marines, les cires de polyéthylène ou de polyoléfines.

10. Composition selon l'une quelconque des revendications 1 à 6, **caractérisée par le fait que** les composés de type céramides sont choisis parmi :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

11. Composition selon l'une quelconque des revendications précédentes, **caractérisée en ce que** l'agent conditionneur est présent à une concentration comprise entre 0,001 % et 20 % en poids par rapport au poids total de la composition, de préférence entre 0,01 % et 10 % en poids.

12. Composition selon l'une quelconque des revendications précédentes, **caractérisée par le fait qu'**elle comprend en outre au moins un agent tensioactif choisi parmi les tensioactifs anioniques, non ioniques, amphotères et leurs mélanges.

13. Composition selon la revendication 12, **caractérisée par le fait que** le ou les agents tensioactifs sont présents à une concentration comprise entre 0,1% et 60% en poids, de préférence entre 3% et 40% en poids, et encore plus préférentiellement entre 5% et 30% en poids, par rapport au poids total de la composition.

14. Composition selon l'une quelconque des revendications 1 à 13, **caractérisée par le fait qu'**elle comprend en outre au moins un agent tensioactif cationique.

15. Composition selon la revendication 14, **caractérisée par** le fait le tensioactif cationique est présent dans des concentrations allant de 0,1 à 10% en poids par rapport au poids total de la composition.

16. Composition selon l'une quelconque des revendications 1 à 15, **caractérisée par** le fait la composition contenir au moins un additif choisi parmi les épaississants, les parfums, les agents nacrants, les conservateurs, les filtres solaires siliconés ou non, les vitamines, les provitamines, les polymères cationiques, amphotères, anioniques ou non ioniques, les protéines, les hydrolysats de protéine, l'acide méthyl-18 eicosanoique, les hydroxyacides, le panthénol, les silicones volatiles ou non volatiles, cycliques ou linéaires ou réticulés, modifiées ou non.

17. Composition selon l'une quelconque des revendications précédentes, **caractérisée par le fait qu'**elle se présente sous forme de shampooing, d'après-shampooing à rincer ou non, de composition pour la permanente, le défrisage, la coloration ou la décoloration des cheveux, de composition à rincer à appliquer entre les deux étapes d'une permanente ou d'un défrisage, de composition lavantes pour le corps.

18. Utilisation d'une composition telle que définie dans l'une quelconque des revendications précédentes pour le lavage ou pour le soin des matières kératiniques.

19. Procédé de traitement des matières kératiniques, telles que les cheveux, **caractérisé en ce qu'**il consiste à appliquer sur lesdites matières une composition cosmétique selon l'une des revendications 1 à 17, puis à effectuer éventuellement un rinçage à l'eau.

20. Utilisation d'un copolymère siliconé tel que défini à l'une des revendications 1 à 5 dans, ou pour la fabrication d'une composition cosmétique comprenant un agent conditionneur choisi parmi .
- les huiles de synthèse
- les huiles animales ou végétales et
- les huiles fluorées ou perfluorées,
- les cires naturelles ou synthétiques
- les céramides de formule (I) suivante : 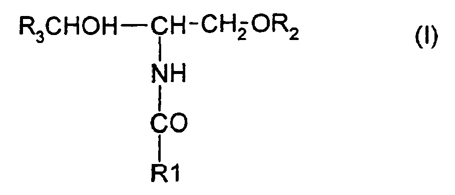 dans laquelle :
- R₁ désigne un radical alkyle, linéaire ou ramifié, saturé ou insaturé, dérivé d'acides gras en C₁₄-C₃₀, ce radical pouvant être substitué par un groupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un radical hydrocarboné en C₁₅-C₂₆, saturé ou insaturé en position α , ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀.

## Claims

1. Cosmetic composition, **characterized in that** it comprises, in a cosmetically acceptable medium, at least one conditioner chosen from:
- synthetic oils
- animal or plant oils
- fluoro oils or perfluoro oils
- natural or synthetic waxes
- the ceramides of formula (I) below: 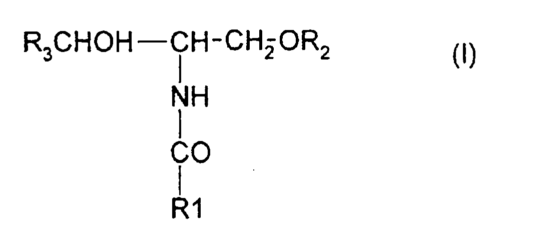 in which:
- R₁ denotes a linear or branched, saturated or unsaturated alkyl radical derived from C₁₄-C₃₀ fatty acids, it being possible for this radical to be substituted with a hydroxyl group in the α position, or a hydroxyl group in the ω position esterified with a saturated or unsaturated C₁₆-C₃₀ fatty acid;
- R₂ denotes a hydrogen atom or a (glycosyl)ₙ, (galactosyl)ₘ or sulphogalactosyl radical, in which n is an integer ranging from 1 to 4 and m is an integer ranging from 1 to 8;
- R₃ denotes a C₁₅-C₂₆ hydrocarbon-based radical saturated or unsaturated in the α position, it being possible for this radical to be substituted with one or more C₁-C₁₄ alkyl radicals;
it being understood that, in the case of the natural ceramides or glycoceramides, R₃ can also denote a C₁₅-C₂₆ α-hydroxyalkyl radical, the hydroxyl radical optionally being esterified with a C₁₆-C₃₀ α-hydroxy acid,
and
at least one silicone copolymer with a viscosity of between 10⁶ and 100 x 10⁶ cP, resulting from the addition reaction, in the presence of a catalyst, of at least:
- (a) one polysiloxane of formula (I) : 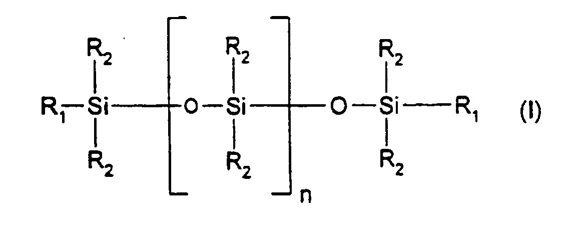 in which:
R₁ denotes a group which can react by chain addition reaction such as, for example, a hydrogen atom or an aliphatic group containing an ethylenic unsaturation, in particular vinyl, allyl or hexenyl;
the groups R₂ in formula (I) represent alkyl groups containing from 1 to 20 carbon atoms, cycloalkyl groups containing from 5 to 6 carbon atoms, aryl groups, alkylaryl groups containing from 7 to 20 carbon atoms or hydroxyl groups and can also comprise functional groups such as ethers, amines, carboxyls, hydroxyls, thiols, esters, sulphonates or sulphates,
n is an integer such that the polysiloxane of formula (I) preferably has a kinetic viscosity of between 1 and 1 × 10⁶ mm²/s.
- (b) and of at least one silicone compound comprising at least one and not more than two groups capable of reacting with the groups R₁ of the polysiloxane (a), at least one of the compounds of type (a) or (b) contains an aliphatic group containing an ethylenic unsaturation.

2. Composition according to Claim 1, **characterized in that** R₂ denotes methyl.

3. Composition according to either of Claims 1 and 2, **characterized in that** the compound of type (b) is another polysiloxane of type (a) in which the groups R₁ of the polysiloxane (b) can react with the groups R₁ of the polysiloxane (a).

4. Composition according to any one of Claims 1 to 3, **characterized in that** the silicone copolymer is obtained by addition reaction, in the presence of a hydrosilylation catalyst, of at least:
- (a) one α,ω-divinylpolydimethylsiloxane, and
- (b) one α,ω-dihydrogenopolydimethylsiloxane.

5. Composition according to any one of Claims 1 to 4, **characterized in that** the silicone copolymer is in the form of an aqueous emulsion.

6. Composition according to any one of Claims 1 to 5, **characterized in that** the silicone copolymer is present at a concentration of between 0.05% and 10% by weight relative to the total weight of the composition.

7. Composition according to any one of Claims 1 to 6, **characterized in that** the synthetic oils are polyolefins of hydrogenated or non-hydrogenated polybutene type or of hydrogenated or non-hydrogenated polydecene type.

8. Composition according to any one of Claims 1 to 6, **characterized in that** the animal or plant oils are chosen from the group formed by sunflower oil, corn oil, soybean oil, avocado oil, jojoba oil, marrow oil, grapeseed oil, sesame oil, hazelnut oil, fish oils, glyceryl tricaprocaprylate, or plant or animal oils of formula R₉COOR₁₀ in which R₉ represents a higher fatty acid residue containing from 7 to 29 carbon atoms and R₁₀ represents a linear or branched hydrocarbon-based chain containing from 3 to 30 carbon atoms, in particular alkyl or alkenyl, natural or synthetic essential oils such as, for example, eucalyptus oil, hybrid lavender oil, lavender oil, vetiver oil, Litsea cubeba oil, lemon oil, sandalwood oil, rosemary oil, camomile oil, savory oil, nutmeg oil, cinnamon oil, hyssop oil, caraway oil, orange oil, geraniol oil, cade oil and bergamot oil.

9. Composition according to any one of Claims 1 to 6, **characterized in that** the wax(es) is(are) chosen from carnauba wax, candelilla wax, alfalfa wax, paraffin wax, ozokerite, plant waxes such as olive tree wax, rice wax, hydrogenated jojoba wax or absolute waxes of flowers such as the essential wax of blackcurrant flower, animal waxes such as beeswaxes, marine waxes, and polyethylene waxes or polyolefin waxes.

10. Composition according to any one of Claims 1 to 6, **characterized in that** the ceramide-type compounds are chosen from:
- 2-N-linoleylaminooctadecane-1,3-diol,
- 2-N-oleylaminooctadecane-1,3-diol,
- 2-N-palmitoylaminooctadecane-1,3-diol,
- 2-N-stearylaminooctadecane-1,3-diol,
- 2-N-behenylaminooctadecane-1,3-diol,
- 2-N-[2-hydroxypalmitoyl]aminooctadecane-1,3-diol,
- 2-N-stearylaminooctadecane-1,3,4-triol and in particular N-stearylphytosphingosine,
- 2-N-palmitoylaminohexadecane-1,3-diol or mixtures of these compounds.

11. Composition according to any one of the preceding claims, **characterized in that** the conditioner is present at a concentration of between 0.001% and 20% by weight relative to the total weight of the composition, preferably between 0.01% and 10% by weight.

12. Composition according to any one of the preceding claims, **characterized in that** it also comprises at least one surfactant chosen from anionic, nonionic and amphoteric surfactants, and mixtures thereof.

13. Composition according to Claim 12, **characterized in that** the surfactant(s) is(are) present at a concentration of between 0.1% and 60% by weight, preferably between 3% and 40% by weight and even more preferably between 5% and 30% by weight, relative to the total weight of the composition.

14. Composition according to any one of Claims 1 to 13, **characterized in that** it also comprises at least one cationic surfactant.

15. Composition according to Claim 14, **characterized in that** the cationic surfactant is present in concentrations ranging from 0.1% to 10% by weight relative to the total weight of the composition.

16. Composition according to any one of Claims 1 to 15, **characterized in that** it contains at least one additive chosen from thickeners, fragrances, nacreous agents, preserving agents, silicone or non-silicone sunscreens, vitamins, provitamins, cationic, amphoteric, anionic or nonionic polymers, proteins, protein hydrolysates, 18-methyleicosanoic acid, hydroxy acids, panthenol and volatile or non-volatile, cyclic or linear or crosslinked, modified or non-modified silicones.

17. Composition according to any one of the preceding claims, **characterized in that** it is in the form of a shampoo, a rinse-out or leave-in conditioner, a composition for permanent-waving, straightening, dyeing or bleaching the hair, a rinse-out composition to be applied between the two steps of a permanent-waving or hair-straightening operation, or washing compositions for the body.

18. Use of a composition as defined in any one of the preceding claims, for washing or caring for keratin materials.

19. Process for treating keratin materials, such as the hair, **characterized in that** it consists in applying a cosmetic composition according to one of Claims 1 to 17 to the said keratin materials, and then in optionally rinsing it out with water.

20. Use of a silicone copolymer, as defined in one of Claims 1 to 5, in, or for the manufacture of, a cosmetic composition comprising a conditioner chosen from:
- synthetic oils
- animal or plant oils
- fluoro oils or perfluoro oils
- natural or synthetic waxes
- the ceramides of formula (I) below: 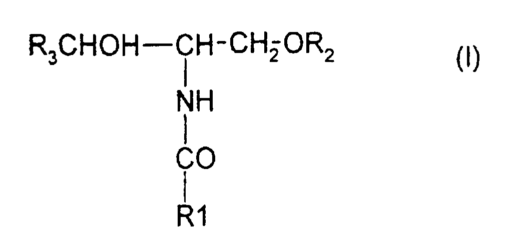 in which:
- R₁ denotes a linear or branched, saturated or unsaturated alkyl radical derived from a C₁₄-C₃₀ fatty acid, it being possible for this radical to be substituted with a hydroxyl group in the α position, or a hydroxyl group in the ω position esterified with a saturated or unsaturated C₁₆-C₃₀ fatty acid;
- R₂ denotes a hydrogen atom or a (glycosyl)ₙ, (galactosyl)ₘ or sulphogalactosyl radical, in which n is an integer ranging from 1 to 4 and m is an integer ranging from 1 to 8;
- R₃ denotes a C₁₅-C₂₆ hydrocarbon-based radical saturated or unsaturated in the α position, it being possible for this radical to be substituted with one or more C₁-C₁₄ alkyl radicals; it being understood that, in the case of the natural ceramides or glycoceramides, R₃ can also denote a C₁₅-C₂₆ α-hydroxyalkyl radical, the hydroxyl group optionally being esterified with a C₁₆-C₃₀ α-hydroxy acid.

## Patentansprüche

1. Kosmetische Zusammensetzung, **dadurch gekennzeichnet, dass** sie in einem kosmetisch akzeptablen Medium mindestens ein Konditioniermittel, das ausgewählt ist unter:
- synthetischen Ölen;
- tierischen oder pflanzlichen Ölen;
- fluorierten oder perfluorierten Ölen;
- natürlichen oder synthetischen Wachsen;
- Ceramiden der folgenden Formel (I): 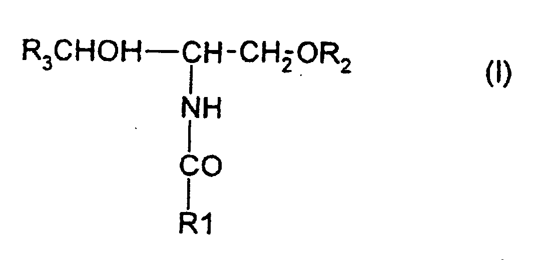
worin bedeuten:
- R₁ eine gesättigte oder ungesättigte, geradkettige oder verzweigte Alkylgruppe, die von C₁₄₋₃₀-Fettsäuren abgeleitet ist,
wobei diese Gruppe gegebenenfalls in α-Stellung mit einer Hydroxygruppe substituiert sein kann, oder einer Hydroxygruppe in ω-Stellung, die mit einer gesättigten oder ungesättigten C₁₆₋₃₀-Fettsäure verestert ist;
- R₂ ein Wasserstoffatom, (Glycosyl)ₙ, (Galactosyl)ₘ oder Sulfogalactosyl, wobei n eine ganze Zahl von 1 bis 4 und m eine ganze Zahl von 1 bis 8 bedeutet;
- R₃ eine gesättigte oder in α-Stellung ungesättigte C₁₅₋₂₆₋Kohlenwasserstoffgruppe, wobei diese Gruppe mit einer oder mehreren C₁₋₁₄-Alkylgruppen substituiert sein kann;
mit der Maßgabe, dass im Falle von natürlichen Ceramiden oder Glycoceramiden R₃ auch eine C₁₅₋₂₆-α-Hydroxyalkylgruppe bedeuten kann, wobei die Hydroxygruppe gegebenenfalls mit einer C₁₆₋₃₀-α-Hydroxysäure verestert sein kann;
und mindestens ein Siliconcopolymer mit einer Viskosität von 10⁶ bis 100·10⁶ cP enthält, entstehend in Gegenwart eines Katalysators bei der Addition von zumindest:
(a) einem Polysiloxan der Formel (I): 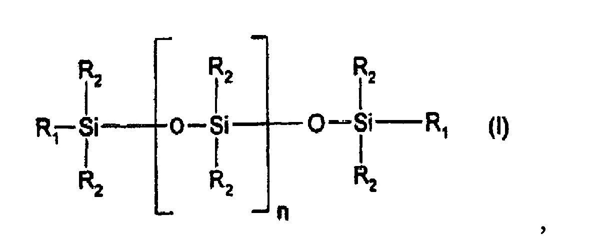 worin bedeuten:
R1 eine Gruppe, die durch Kettenaddition reagieren kann, wie beispielsweise ein Wasserstoffatom, eine aliphatische ethylenisch ungesättigte Gruppe, besonders Vinyl, Allyl oder Hexenyl,
wobei die Gruppen R2 der Formel (I) Alkylgruppen mit 1 bis 20 Kohlenstoffatomen, Cycloalkylgruppen mit 5 oder 6 Kohlenstoffatomen, Arylgruppen, Alkylarylgruppen mit 7 bis 20 Kohlenstoffatomen oder Hydroxy bedeuten und ferner funktionelle Gruppen enthalten können, wie beispielsweise Ethergruppen, Amingruppen, Carboxygruppen, Hydroxygruppen, Thiolgruppen, Estergruppen, Sulfonatgruppen, Sulfatgruppen,
n eine ganze Zahl, die so gewählt ist, dass das Polysiloxan der Formel (I) vorzugsweise eine kinematische Viskosität von 1 bis 1·10⁶ mm²/s aufweist;
(b) und mindestens einer Silicon-haltigen Verbindung, die mindestens eine und höchstens zwei Gruppen aufweist, die mit den Gruppen R1 des Polysiloxans (a) reagieren können,
wobei mindestens eine der Verbindungen vom Typ (a) oder (b) eine aliphatische Gruppe enthält, die ethylenisch ungesättigt ist.

2. Zusammensetzung nach Anspruch 1, **dadurch gekennzeichnet, dass** R2 Methyl bedeutet.

3. Zusammensetzung nach einem der Ansprüche 1 oder 2, **dadurch gekennzeichnet, dass** die Verbindung vom Typ (b) ein anderes Polysiloxan vom Typ (a) ist, wobei die Gruppen R1 des Polysiloxans (b) mit den Gruppen R1 des Polysiloxans (a) reagieren können.

4. Zusammensetzung nach einem der Ansprüche 1 bis 3, **dadurch gekennzeichnet, dass** das Siliconcopolymer in Gegenwart eines Hydrosilylierungskatalysators durch Addition hergestellt wird von zumindest:
(a) einem alpha,omega-Divinylpolydimethylsiloxan und
(b) einem alpha,omega-Dihydrogenopolydimethylsiloxan.

5. Zusammensetzung nach einem der Ansprüche 1 bis 4, **dadurch gekennzeichnet, dass** das Siliconcopolymer in Form einer wässerigen Emulsion vorliegt.

6. Zusammensetzung nach einem der Ansprüche 1 bis 5, **dadurch gekennzeichnet, dass** das Siliconcopolymer in einer Konzentration von 0,05 bis 10 Gew.-%, bezogen auf das Gesamtgewicht der Zusammensetzung, enthalten ist.

7. Zusammensetzung nach einem der Ansprüche 1 bis 6, **dadurch gekennzeichnet, dass** die synthetischen Öle Polyolefine vom Typ der hydrierten oder nicht hydrierten Polybutene oder vom Typ der hydrierten oder nicht hydrierten Polydecene sind.

8. Zusammensetzung nach einem der Ansprüche 1 bis 6, **dadurch gekennzeichnet, dass** die tierischen oder pflanzlichen Öle unter Sonnenblumenöl, Maisöl, Sojaöl, Avocadoöl, Jojobaöl, Kürbiskernöl, Traubenkernöl, Sesamöl, Haselnussöl, Fischölen, Glycerintricaprocaprylat, pflanzlichen oder tierischen Ölen der Formel R₉COOR₁₀, worin Rg den Rest einer höheren Fettsäure mit 7 bis 29 Kohlenstoffatomen und R₁₀ eine geradkettige oder verzweigte Kohlenwasserstoffkette mit 3 bis 20 Kohlenstoffatomen und insbesondere Alkyl oder Alkenyl bedeutet, natürlichen oder synthetischen etherischen Ölen, wie beispielsweise Eukalyptusöl, Lavandinöl, Lavendelöl, Vetiveröl, Öl aus Litsea cubeba, Citronenöl, Sandelholzöl, Rosmarinöl, Kamilleöl, Pfefferkrautöl, Muskatnussöl, Zimtöl, Ysopöl, Kümmelöl, Orangenöl, Geraniol, Kadeöl und Bergamotteöl, ausgewählt sind.

9. Zusammensetzung nach einem der Ansprüche 1 bis 6, **dadurch gekennzeichnet, dass** das Wachs oder die Wachse unter Carnaubawachs, Candelillawachs, Alfawachs, Paraffinwachs, Ozokerit, pflanzlichen Wachsen, wie Wachs von Ölbäumen, Reiswachs, hydriertem Jojobawachs oder absoluten Blütenwachsen, wie dem etherischen Wachs von Blüten der Schwarzen Johannisbeere, tierischen Wachsen, wie Bienenwachs, marinen Wachsen, Polyethylenwachsen oder Polyolefinwachsen ausgewählt sind.

10. Zusammensetzung nach einem der Ansprüche 1 bis 6, **dadurch gekennzeichnet, dass** die Verbindungen vom Ceramidtyp ausgewählt sind unter:
- 2-N-Linoleoylamino-octadecan-1,3-diol,
- 2-N-Oleoylamino-octadecan- 1,3-diol,
- 2-N-Palmitoylamino-octadecan-1,3-diol,
- 2-N-Stearoylamino-octadecan-1,3-diol,
- 2-N-Behenoylamino-octadecan-1,3-diol,
- 2-N-[2-Hydroxy-palmitoyl]-amino-octadecan-1,3-diol,
- 2-N-Stearoylamino-octadecan-1,3,4-triol und insbesondere N-Stearoyl-phytosphingosin,
- 2-N-Palmitoylamino-hexadecan-1,3-diol,
- oder Gemischen dieser Verbindungen.

11. Zusammensetzung nach einem der vorhergehenden Ansprüche, **dadurch gekennzeichnet, dass** das Konditioniermittel in einer Konzentration von 0,001 bis 20 Gew.-%, bezogen auf das Gesamtgewicht der Zusammensetzung, und vorzugsweise 0,01 bis 10 Gew.-%, enthalten ist.

12. Zusammensetzung nach einem der vorhergehenden Ansprüche, **dadurch gekennzeichnet, dass** sie ferner mindestens einen grenzflächenaktiven Stoff enthält, der unter den anionischen, nichtionischen oder amphoteren grenzflächenaktiven Stoffen und deren Gemischen ausgewählt ist.

13. Zusammensetzung nach Anspruch 12, **dadurch gekennzeichnet, dass** der oder die grenzflächenaktive(n) Stoff(e) in einer Konzentration von 0,1 bis 60 Gew.-%, vorzugsweise 3 bis 40 Gew.-% und noch bevorzugter 5 bis 30 Gew.-%, bezogen auf das Gesamtgewicht der Zusammensetzung, enthalten sind.

14. Zusammensetzung nach einem der vorhergehenden Ansprüche 1 bis 13, **dadurch gekennzeichnet, dass** sie ferner mindestens einen kationischen grenzflächenaktiven Stoff enthält.

15. Zusammensetzung nach Anspruch 14, **dadurch gekennzeichnet, dass** der kationische grenzflächenaktive Stoff in Konzentrationen von 0,1 bis 10 Gew.-%, bezogen auf das Gesamtgewicht der Zusammensetzung, enthalten ist.

16. Zusammensetzung nach einem der Ansprüche 1 bis 15, **dadurch gekennzeichnet, dass** die Zusammensetzung mindestens einen Zusatzstoff enthält, der unter den Verdickungsmitteln, Parfums, Perlglanzstoffen, Konservierungsmitteln, Sonnenschutzfiltern, die gegebenenfalls siliconhaltig sind, Vitaminen, Provitaminen, kationischen Polymeren, amphoteren Polymeren, anionischen Polymeren oder nichtionischen Polymeren, Proteinen, Proteinhydrolysaten, 18-Methyleicosansäure, Hydroxysäuren, Panthenol, cyclischen oder linearen oder vernetzten, gegebenenfalls modifizierten, flüchtigen oder nicht flüchtigen Siliconen ausgewählt ist.

17. Zusammensetzung nach einem der vorhergehenden Ansprüche, **dadurch gekennzeichnet, dass** sie als Haarwaschmittel, Conditioner, der ausgespült wird oder im Haar verbleibt, Zusammensetzung für dauerhafte Verformungen, Entkräuselungen, Färbungen oder Entfärbungen der Haare, Zusammensetzung, die ausgespült wird und zwischen den beiden Schritten einer permanenten Verformung oder Entkräuselung aufgebracht wird oder reinigende Zusammensetzung für den Körper vorliegt.

18. Verwendung einer Zusammensetzung nach einem der vorhergehenden Ansprüche zur Reinigung oder zur Pflege von Keratinsubstanzen.

19. Verfahren zur Behandlung von Keratinsubstanzen, beispielsweise zur Behandlung der Haare, das **dadurch gekennzeichnet ist, dass** es darin besteht, auf diese Substanzen eine kosmetische Zusammensetzung nach einem der Ansprüche 1 bis 17 aufzutragen und gegebenenfalls mit Wasser zu spülen.

20. Verwendung eines Siliconcopolymers nach einem der Ansprüche 1 bis 5 in einer kosmetischen Zusammensetzung oder für die Herstellung einer solchen Zusammensetzung, die ein Konditioniermittel enthält, das ausgewählt ist unter:
- synthetischen Ölen;
- tierischen oder pflanzlichen Ölen;
- fluorierten oder perfluorierten Ölen;
- natürlichen oder synthetischen Wachsen;
- Ceramiden der folgenden Formel (I): 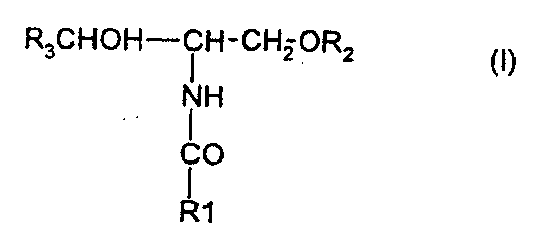
worin bedeuten:
- R₁ eine gesättigte oder ungesättigte, geradkettige oder verzweigte Alkylgruppe, die von C₁₄₋₃₀-Fettsäuren abgeleitet ist,
wobei diese Gruppe gegebenenfalls in α-Stellung mit einer Hydroxygruppe substituiert sein kann, oder einer Hydroxygruppe in ω-Stellung, die mit einer gesättigten oder ungesättigten C₁₆₋₃₀-Fettsäure verestert ist;
- R₂ ein Wasserstoffatom, (Glycosyl)ₙ, (Galactosyl)ₘ oder Sulfogalactosyl, wobei n eine ganze Zahl von 1 bis 4 und m eine ganze Zahl von 1 bis 8 bedeutet;
- R₃ eine gesättigte oder in α-Stellung ungesättigte C₁₅₋₂₆₋Kohlenwasserstoffgruppe, wobei diese Gruppe mit einer oder mehreren C₁₋₁₄-Alkylgruppen substituiert sein kann;
mit der Maßgabe, dass im Falle von natürlichen Ceramiden und Glycoceramiden R₃ auch eine C₁₅₋₂₆-α-Hydroxyalkylgruppe bedeuten kann, wobei die Hydroxygruppe gegebenenfalls mit einer c₁₆₋₃₀-α-Hydroxysäure verestert sein kann.
